# EUROPEAN PATENT SPECIFICATION

(11) **EP 2 336 346 B1**
(45) Date of publication and mention of the grant of the patent: **07.12.2016**
(21) Application number: 09817864.3
(22) Date of filing: 01.10.2009
(51) Int. Cl.: C12M 1/02, C12P 13/02

(54) **METHOD FOR PRODUCING ACRYLAMIDE**
VERFAHREN ZUR HERSTELLUNG VON ACRYLAMID
PROCÉDÉ DE PRODUCTION D'ACRYLAMIDE

(30) Priority: 03.10.2008 JP 2008258240
(43) Date of publication of application: 22.06.2011
(73) Proprietor: Dia-Nitrix Co., Ltd., Minato-ku Tokyo 105-0014 (JP)
(72) Inventor: KANO, Makoto, Yokohama-shi Kanagawa 230-0053 (JP); NIWA, Kiyonobu, Yokohama-shi Kanagawa 230-0053 (JP); MURAO, Kozo, Yokohama-shi Kanagawa 230-0053 (JP)
(74) Representative: Hoffmann Eitle
(86) International application number: PCT/JP2009/067175
(87) International publication number: WO 2010/038832

(56) References cited:
- EP-A2- 0 231 944
- WO-A1-02/088373
- WO-A1-03/000914
- JP-T- 2004 524 047
- JP-T- 2005 507 643

## Description

### TECHNICAL FIELD

The present invention relates to a method for producing acrylamide from acrylonitrile using a biocatalyst.

### BACKGROUND ART

A method for producing a compound of interest utilizing a biocatalyst has advantages that reaction conditions are mild, that the purity of a reaction product is high with a small amount of a by-product, and that a production process can be simplified. Therefore, such a biocatalyst is used for many compounds to be produced. In the case of production of an amide compound, since nitrile hydratase, which is an enzyme for converting a nitrile compound into an amide compound, was found, biocatalysts including the enzyme have been widely used.

In order to industrially utilize such a biocatalyst, producing an amide compound at low cost is essential. In general, a biocatalyst is easily deactivated with respect to heat. Therefore, if a reaction heat is not sufficiently removed, the supply amount of a catalyst is increased and the catalyst cost is increased. In addition, energy costs are increased because, for example, the temperature of cooling water for removing heat is decreased and the supply amount of cooling water is increased. As a result, it becomes difficult to produce acrylamide at low cost.

As methods for removing reaction heat, for example, a method for cooling in which a reaction solution is circulated in a heat exchanger provided to the exterior portion of a reactor (Patent Document 1), a method of using a double pipe type or a shell and tube type in a plug flow type reactor (Patent Document 2) and a method in which a reaction tank is equipped with a jacket or a cooling coil (Patent Document 3) are known.

### PRIOR ART DOCUMENTS

### [Patent Documents]

[Patent Document 1] Japanese National-phase PCT Laid-Open Patent Publication No. 2004-524047
[Patent Document 2] Japanese Laid-Open Patent Publication No. 2001-340091
[Patent Document 3] International Publication WO 03/00914 pamphlet

### DISCLOSURE OF THE INVENTION

### PROBLEMS TO BE SOLVED BY THE INVENTION

However, when using the method described in the above-described Patent Document 1 (Japanese National-phase PCT Laid-Open Patent Publication No. 2004-524047), costs for facilities are high due to complicated apparatuses, and in addition, a great deal of energy is expended for circulating a reaction solution. As a result, production costs are increased. Further, when using the methods described in the above-described Patent Documents 2 and 3 (Japanese Laid-Open Patent Publication No. 2001-340091; International Publication WO 03/00914 pamphlet), if the productivity per reactor is increased, an oversize heat exchanger is required, and economical load of facilities is increased. Moreover, these methods have been examined with respect to the case of small-scale reactors, and effects of heat removal on industrial scale have not been considered. In the case where a heat exchanger is provided to the inside of a reactor, in view of industrial scale-up, the larger the volume of the reactor is, the smaller the ratio of a heat-transfer area used for cooling per volume is, and therefore, it is more difficult to remove heat.

Therefore, the purpose of the present invention is to provide a method for producing acrylamide from acrylonitrile using a biocatalyst with high productivity at low cost, in which reaction heat can be efficiently removed even at an industrial reaction scale.

### MEANS FOR SOLVING THE PROBLEMS

The present inventor diligently made researches in order to solve the above-described problem. As a result, the present inventor found that the above-described problem can be solved by using an industrial-scale reactor which is in the inside equipped with a tubular heat exchanger as a reactor and maintaining the difference between a reaction temperature and a temperature of a cooling water to be introduced into the heat exchanger within a certain range, and thus the present invention was achieved.

Specifically, the present invention is as described below.

A method for producing acrylamide by reacting acrylonitrile in the presence of a biocatalyst, wherein the reaction is performed using a reactor having a reaction volume of 1 m³ or more equipped with a tubular heat exchanger in the inside of said reactor, and wherein the difference between a temperature of a cooling water to be introduced into the heat exchanger and a reaction temperature is maintained from 5 to 20°C.

In the method of the present invention, examples of the reaction include a continuous reaction. Further, the continuous reaction can be performed, for example, with a value that is obtained by dividing the difference between an acrylamide concentration in a reaction mixture at an inlet of the reactor and that at an outlet of the reactor (wt%) by the difference between the temperature of the cooling water and the reaction temperature (°C) (wt%/°C) being maintained within a range of 1.5 to 2.5.

Moreover, in the method of the present invention, as the tubular heat exchanger, a multitubular heat exchanger, a double or more coil type heat exchanger or the like can be used. As the multitubular heat exchanger, for example, a U-tube type heat exchanger can be used. Examples of the multitubular heat exchanger (particularly U-tube type heat exchanger) include those having 2 to 6 flow paths, those installed upright in the reactor, and those having a structure in which a tube bundle is detachable.

### ADVANTAGEOUS EFFECT OF THE INVENTION

According to the present invention, at the time of producing acrylamide from acrylonitrile using a biocatalyst, reaction heat can be efficiently removed in an industrial-scale reactor, and it is possible to provide a method for producing acrylamide with high productivity at low cost.

### BRIEF DESCRIPTION OF THE DRAWINGS

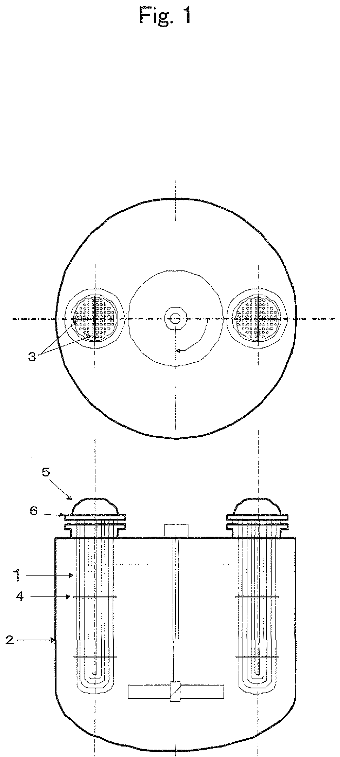

FIG. 1 shows an example of the reactor of the present invention in which multitubular heat exchangers (U-tube type) which efficiently remove reaction heat are disposed.

### BEST MODE FOR CARRYING OUT THE INVENTION

Hereinafter, the method for producing acrylamide of the present invention will be described in detail. The scope of the present invention is not limited to the description. In addition to the following examples, the present invention can be suitably changed and then practiced within a range in which the effects of the present invention are not reduced.

As described above, the method for producing acrylamide of the present invention is as defined in claim 1.

Examples of the biocatalyst used in the method of the present invention include an animal cell, plant cell, organelle, bacterial cell (living cell or dead cell), and treated products thereof, all of which contain an enzyme for catalyzing a reaction of interest. Examples of the treated products include a crude enzyme or purified enzyme extracted from a cell, and a product in which an animal cell, plant cell, organelle, bacterial cell (living cell or dead cell) or enzyme itself is immobilized using the entrapping method, cross-linking method, carrier binding method or the like. In this regard, the entrapping method is a method in which a bacterial cell or enzyme is enclosed with a fine lattice of polymer gel or coated with a semipermeable polymer membrane. The cross-linking method is a method in which an enzyme is crosslinked with a reagent having 2 or more functional groups (polyfunctional crosslinking agent). The carrier binding method is a method in which an enzyme is bound to a water-insoluble carrier.

Examples of immobilization carriers to be used for immobilization include glass beads, silica gel, polyurethane, polyacrylamide, polyvinyl alcohol, carrageenan, alginic acid, agar and gelatin.

Examples of the bacterial cells include microorganisms belonging to Nocardia, Corynebacterium, Bacillus, Pseudomonas, Micrococcus, Rhodococcus, Acinetobacter, Xanthobacter, Streptomyces, Rhizobium, Klebsiella, Enterobavter, Erwinia, Aeromonas, Citrobacter, Achromobacter, Agrobacterium and Pseudonocardia.

Examples of enzymes include nitrile hydratase produced by the aforementioned microorganisms.

The amount of the biocatalyst to be used varies depending on the type and form of the biocatalyst to be used, but it is preferably adjusted so that the activity of the biocatalyst added to a reactor becomes about 50 to 200 U per 1 mg of dried cell at reaction temperature of 10°C. Note that the aforementioned unit "U" means an amount of the enzyme by which 1 micromole of acrylamide is produced from acrylonitrile for 1 minute, and the value is measured using acrylonitrile to be used in the production.

In the production method of the present invention, any of the following reactions may be applied: (i) a method in which the total amount of reaction raw materials (including a biocatalyst, acrylonitrile and raw material water) are fed into a reactor at a time and then a reaction is performed (batch reaction); (ii) a method in which a part of reaction raw materials are fed into a reactor and then remaining reaction raw materials are fed continuously or intermittently and a reaction is performed (semibatch reaction); and (iii) a method of continuous production by continuously or intermittently supplying reaction raw materials and continuously or intermittently taking out a reaction mixture (including reaction raw materials and acrylamide produced) without taking out the total amount of the reaction mixture in the reactor (continuous reaction). Since it is easy to produce a large amount of acrylamide industrially and efficiently, continuous reaction is preferred. Further, one reactor or a plurality of reactors may be used. When continuously performing a reaction using a plurality of reactors, the reactor into which the biocatalyst and acrylonitrile are to be supplied is not limited to the most upstream reactor, and the materials may also be introduced into a reactor downstream thereof, as long as it is within a range in which efficiency of the reaction, etc. are not reduced too much. The reaction temperature (reaction mixture temperature) in the production method of the present invention is not limited, but is preferably 10 to 40°C, and more preferably 20 to 35°C. When the reaction temperature is 10°C or higher, not only the reaction activity of the biocatalyst can be sufficiently increased, but also the temperature of cooling water can be increased. Therefore, a cooling tower can be utilized instead of a refrigerating machine, and cooling energy of cooling water can be reduced. Further, when the reaction temperature is 40°C or lower, it becomes easier to suppress deactivation of the biocatalyst.

Further, the reaction time in the production method of the present invention is not limited, but is, for example, preferably 1 to 50 hours, and more preferably 3 to 20 hours.

Preferred examples of the tubular heat exchanger to be used in the production method of the present invention include a multitubular heat exchanger and a double or more coil type heat exchanger.

The above-described multitubular heat exchanger means a heat exchanger having a structure in which many heat-transfer tubes are fixed to a tube plate by means of welding or the like. It is a heat exchanger having a structure in which cooling water is introduced into the inside of each of the heat-transfer tubes and heat exchange with the reaction mixture is performed via walls of the heat-transfer tubes. The arrangement method for the heat-transfer tubes is not particularly limited, and examples thereof include a method of triangular arrangement and a method of quadrangular arrangement. Further, in the arrangement of the heat-transfer tubes, the center distance between two adjacent tubes is preferably 1 to 5 times, and more preferably 1.25 to 3 times the outer diameter of the tube. The flow rate of cooling water in the heat-transfer tube is preferably 0.5 to 5 m/s, and more preferably 1 to 3 m/s.

Preferred examples of the multitubular heat exchanger include a U-tube type heat exchanger and a straight tube type heat exchanger. Among them, a U-tube type heat exchanger is more preferred.

The U-tube type heat exchanger is a heat exchanger in which heat-transfer tubes are bent into the U shape and tube ends are attached to a tube plate. The minimum bending radius of the U-tube is preferably 1 to 3 times, and more preferably 1.5 to 2.5 times the outer diameter of the heat-transfer tube.

In the U-tube type heat exchanger, since tube ends are fixed to one tube plate, expansion and contraction of heat-transfer tubes due to temperature of a fluid becomes flexible, and the combination of the reaction temperature and the temperature of cooling water can be easily set in a wide range. In addition, because of the simple structure, the U-tube type heat exchanger can be produced or obtained relatively inexpensively. Moreover, since a tube bundle can be pulled out for cleaning and checking, the U-tube type heat exchanger provides ease of maintenance.

In the multitubular heat exchanger (particularly the U-tube type heat exchanger) to be used in the present invention, for example, the number of flow paths is preferably 2 to 6, and more preferably 2 to 4. Usually, the inlet/outlet for cooling water of the heat-transfer tube of the heat exchanger has a divided chamber, and to the inside thereof, division plates for introducing cooling water are attached. The number of flow paths means the number of divisions in the divided chamber divided by the division plates (hereinafter referred to as N) (for example, see "Process Equipment Structural Design Series 1 -Heat exchanger-", Society for Chemical Engineers Ed., p. 23 (Maruzen Co., Ltd.)). Specifically, for example, when N is 2, the number of flow paths is 2. When N is 3, the number of flow paths is 4. When N is 4, the number of flow paths is 6. Constitutionally, the number of flow paths of the U-tube type heat exchanger is 2 or more. When the number of flow paths is more than 6, the number of divisions is increased and the structure for heat exchange becomes complicated. As a result, it is impossible to produce or obtain the heat exchanger inexpensively.

In the multitubular heat exchanger (particularly the U-tube type heat exchanger) to be used in the present invention, for example, it is preferred to have a structure in which a tube bundle is detachable. The structure means a structure in which a tube bundle, which is fixed to a reactor, for example, by tightening a flange bolt, can be removed from the reactor. When the tube bundle is detachable, dirt adhered to the heat-transfer tubes can be cleaned easily and safely outside the reactor. The tube bundle means a state in which many heat-transfer tubes and reinforcing materials of the heat-transfer tubes are incorporated into a tube plate.

When the reactor to be used in the production method of the present invention is equipped with the multitubular heat exchanger (in its inside), in the inside of the reactor to which the reaction raw materials are supplied, heat exchange between the reaction mixture and cooling water is performed via the heat-transfer tubes of the tubular heat exchanger. At the time of mixing operation such as stirring, flow of the reaction mixture is disturbed by the multitubular heat exchanger as a barrier, and therefore, the heat-transfer coefficient of the heat-transfer tube is increased. Therefore, more efficient heat exchange can be performed compared to a jacket-type cooling system and a single coil type heat exchanger provided to the inside of the reactor.

Further, when the reactor to be used in the present invention is equipped with the multitubular heat exchanger, it is preferred that the multitubular heat exchanger (particularly the U-tube type heat exchanger) is installed upright in the reactor. In this regard, to be installed upright means a form in which the heat exchanger is disposed in the reactor with the longitudinal direction of the heat-transfer tubes of the heat exchanger being vertical. When the heat exchanger is installed upright, at the time of mixing operation such as stirring for the reaction mixture, the heat exchanger disturbs flow of the reaction mixture, and thereby the heat-transfer coefficient of the outside of the heat-transfer tube (the side in contact with the reaction mixture) is increased. Therefore, the cooling efficiency of the reaction mixture can be improved.

The aforementioned double or more coil type heat exchanger is a heat exchanger in which a tube bundle, in which two or more heat-transfer tubes are wound in a coil-like manner, is provided to the inside of a reactor, and is also called a corrugated tube heat exchanger. A coil usually has a structure in which a tube made of copper, steel, special steel or the like is spirally wound.

The number of coils to be used in the present invention is not particularly limited. It is sufficient when the number is two or more. For example, double or triple is preferred, and double is more preferred. When comparison is made based on the same heat-transfer area, if the number of coils is one (single coil), in order to ensure the heat-transfer area required, the distance between spirally-wound adjacent heat-transfer tubes in the height direction (coil pitch) becomes smaller. Therefore, at the time of mixing operation such as stirring, flow of the reaction mixture at the inside and outside of the spirally-wound coil is suppressed, and as a result, heat exchange cannot be efficiently performed.

Further, when compared to a cooling method using a heat exchanger provided to the outside of a reactor, the present invention can achieve a lower cost because costs for a circulation equipment and pipe arrangement for introducing the reaction mixture to an external heat exchanger and energy costs for driving the circulation equipment are not required.

Regarding the reactor to be used in the present invention, it is sufficient when the reactor is in a form in which a tubular heat exchanger can be placed in the inside of the reactor. Examples thereof include a tank type reactor.

Regarding the reactor to be used in the present invention, the reaction volume is 1 m³ or more, preferably 2 m³ or more, and more preferably 5 m³. In this regard, the reaction volume means a net volume of the reaction mixture that can be introduced (fed) into the reactor. In the case of a multi-tank continuous reactor, the reaction volume means a net volume of the reaction mixture per tank.

In the production method of the preset invention, the cooling water is used for introducing into a tubular heat exchanger (specifically a heat-transfer tube of the heat exchanger) for the purpose of removing reaction heat produced at the time of reaction. The type of the cooling water is not particularly limited, and examples thereof include chloride solutions such as calcium chloride solution, sodium chloride solution and magnesium chloride solution, alcohol solutions such as ethylene glycol solution, and industrial water.

In the production method of the present invention, it is important that the difference between the temperature of the cooling water and the reaction temperature (reaction mixture temperature) (°C) (hereinafter represented by ΔT) is maintained from 5 to 20°C (preferably 4 to 18°C, and more preferably 3 to 15°C) during the reaction. When ΔT is smaller than 5°C, an excessive flow rate of the cooling water and an excessive heat-transfer area of the heat exchanger are required. Therefore, not only costs for equipments such as pumps and pipe arrangement are increased, but also energy costs for pump circulation are increased, and as a result, economical load is increased. Further, when ΔT is more than 20°C, there is a case where the temperature of the cooling water must be decreased to 0°C or lower. As a result, the operation efficiency of a cooling apparatus such as a refrigerating machine is reduced, and energy costs are increased. The method for controlling ΔT is not limited, and for example, it can be controlled by suitably adjusting the flow rate (flow velocity) of the cooling water to be introduced into the heat exchanger. Note that since the temperature of cooling water and the reaction temperature are usually changed at each point on the heat-transfer surface, in the present invention, ΔT means a mean temperature difference. In this regard, as the mean temperature difference, for example, the logarithmic mean temperature difference described in Chemical Engineering Handbook, 6th revised edition, page 392 (published by Maruzen Co., Ltd.) can be used.

In the continuous reaction of the present invention, the reaction is performed with a value that is obtained by dividing the difference between an acrylamide concentration in the reaction mixture at an inlet of the reactor and that at an outlet of the reactor (wt%) (hereinafter represented by ΔC) by ΔT (wt%/°C) (hereinafter represented by ΔC/ΔT) being maintained preferably within a range of 1.5 to 2.5, and more preferably within a range of 1.6 to 2.2. The concentration at the inlet of the reactor means an acrylamide concentration in the reaction mixture flowing from the upstream side of the reaction into the inlet of the reactor. The concentration at the outlet of the reactor means an acrylamide concentration in the reaction mixture flowing out from the outlet of the reactor into the downstream side.

The amount of heat generated by the reaction is proportional to ΔC, and the amount of heat removed by the heat exchanger is proportional to ΔT. When the value of ΔC/ΔT is more than 2.5, since acrylamide productivity per reactor is too high, not only an excessive heat-transfer area is required for removing heat, but also the reaction efficiency is reduced, resulting in increase of a supplied amount of a catalyst. Therefore, it becomes impossible to produce acrylamide inexpensively.

In the production method of the present invention, the rate of conversion from acrylonitrile as the raw material compound to acrylamide as the product of interest is, for example, preferably 85% or higher, more preferably 90% or higher, and even more preferably 95% or higher based on the weight concentration.

In the production method of the present invention, collection and purification of acrylamide after the reaction can be carried out, for example, by concentration operation (evaporative concentration, etc.), activated carbon treatment, ion-exchange treatment, filtration treatment, crystallization operation, etc.

### EXAMPLES

Hereinafter, the present invention will be more specifically described by way of working examples. However, the present invention is not limited only to these examples.

### [Example 1]

### Preparation of biocatalyst,

Rhodococcus rhodochrous J1 having nitrile hydratase activity (Accession number: FERM BP-1478; internationally deposited to International Patent Organism Depositary, National Institute of Advanced Industrial Science and Technology (Chuo 6, Higashi 1-1-1, Tsukuba-shi, Ibaraki) on September 18, 1987) was aerobically cultured in a medium containing 2% glucose, 1% urea, 0.5% peptone, 0.3% yeast extract and 0.05% cobalt chloride (% by mass in each case) (pH 7.0) at 30°C. Using a centrifuge and 50 mM phosphate buffer (pH 7.0), the obtained culture was subjected to harvest and washing, thereby preparing a bacterial cell suspension as a biocatalyst (dried cell: 15% by mass).

### Reaction from acrylonitrile to acrylamide

### (Reaction volume: 2 m³, acrylamide production reaction using a U-tube type heat exchanger, ΔT = 20°C)

Two U-tube type heat exchangers (outer diameter of a heat-transfer tube: 8 mm, the number of heat-transfer tubes: 50, the number of flow paths: 4, heat-transfer area: 3.0 m²) were placed in a reactor with a jacket made of SUS (inner diameter: 1.3 m, height: 2.0 m, bottom face: 1/4 semi-ellipsoid).

To the reactor, 800 L/hr of 50 mM phosphate buffer (pH 7.0), 350 L/hr of acrylonitrile and 2.5 L/hr of the bacterial cell suspension after the above-described preparation were continuously added, and the flow rate of discharging at the outlet of the reactor was controlled so that the liquid amount of the reaction mixture in the reactor became 2 m³.

A cooling water (10% aqueous solution of ethylene glycol) was introduced into the U-tube type heat exchangers, and temperatures of the cooling water were measured using thermometers placed at the inlet side and the outlet side of the heat exchangers. The flow rate of the cooling water was controlled so that the liquid temperature (reaction temperature) in the reactor was maintained at 25°C. Note that the cooling water was not run through the jacket. The temperatures of the cooling water at the inlet and the outlet of the heat exchangers were 3°C and 6.5°C, respectively (ΔT = 20°C).

10 hours after the initiation of the reaction, the reaction solution (25°C) flowing out from the reactor was measured by means of gas chromatography (column: manufactured by Waters, PoraPak-PS, 1 m, 180°C, carrier gas: helium, detector: FID) and a refractometer (ATAGO, RX-1000). Using the calibration curve measured in advance, the acrylonitrile concentration and the acrylamide concentration in the reaction solution were calculated. The acrylonitrile concentration was 1.1 wt%, and the acrylamide concentration was 33.4 wt% (ΔC/ΔT = 1.7). 96% or more of the acrylonitrile supplied to the reactor was converted into acrylamide.

### [Example 2]

### Reaction volume: 2 m³, double coil type heat exchanger, ΔT = 20°C

A reaction from acrylonitrile to acrylamide was performed in a manner similar to that in Example 1, except that the U-tube type heat exchangers were replaced by double coil type heat exchangers (outer diameter of a heat-transfer tube: 34.0 mm, outer coil: coil winding diameter 1.0 m - 10 turns, inner coil: coil winding diameter 0.7 m - 10 turns, heat-transfer area: 6.0 m²).

10 hours after the initiation of the reaction, the reaction solution (25°C) flowing out from the reactor was analyzed by means of gas chromatography and a refractometer. The acrylonitrile concentration was 1.3 wt%, and the acrylamide concentration was 33.2 wt% (ΔC/ΔT = 1.7). 95% or more of the acrylonitrile introduced into the reactor was converted into acrylamide.

### [Comparative Example 1]

### Jacket-type heat exchanger, ΔT = 20°C

A reaction from acrylonitrile to acrylamide was performed in a manner similar to that in Example 2, except that the double coil type heat exchangers were removed from the reactor and cooling water was run through a jacket of the reactor (heat-transfer area: 6.0 m²).

10 hours after the initiation of the reaction, reaction heat was not sufficiently removed because the heat-transfer coefficient in the case of the jacket-type heat exchanger was low. As a result, the heat temperature was increased to 28°C (ΔT ≒ 23°C). The reaction solution flowing out from the reactor was analyzed using gas chromatography and a refractometer. Since the biocatalyst was deactivated due to increase in the temperature, the acrylonitrile concentration became 4.8 wt%, and the acrylamide concentration became 28.5 wt% (ΔC/ΔT = 1.2). Only 82% of the acrylonitrile supplied to the reactor was converted into acrylamide.

### [Comparative Example 2]

### Single coil type heat exchanger, ΔT = 20°C

A reaction from acrylonitrile to acrylamide was performed in a manner similar to that in Example 2, except that single coil type heat exchangers (outer diameter of a heat-transfer tube: 34.0 mm, coil winding diameter: 1.0 m - 18 turns, heat-transfer area: 6.0 m²) were placed in the reactor.

10 hours after the initiation of the reaction, like the case of Comparative Example 1, it was impossible to maintain the reaction temperature at 25°C, and the reaction temperature was increased to 27°C (ΔT ≒ 22°C). The reaction solution flowing out from the reactor was analyzed using gas chromatography and a refractometer. Since the biocatalyst was deactivated due to increase in the temperature, the acrylonitrile concentration became 4.1 wt%, and the acrylamide concentration became 29.4 wt% (ΔC/ΔT = 1.3). Only 84% of the acrylonitrile supplied to the reactor was converted into acrylamide.

### [Example 3]

### Reaction volute: 2 m³, U-tube type heat exchanger, ΔT = 5°C

The same type of U-tube type heat exchangers as Example 1 were used. To a reactor, 1200 L/hr of 50 mM phosphate buffer (pH 7.0), 140 L/hr of acrylonitrile and 0.9 L/hr of the bacterial cell suspension after the above-described preparation were continuously added, and the flow rate of discharging at the outlet of the reactor was controlled so that the liquid amount of the reaction mixture in the reactor became 2 m³.

A cooling water (10% aqueous solution of ethylene glycol) was introduced into the U-tube type heat exchangers, and temperatures of the cooling water were measured using thermometers placed at the inlet side and the outlet side of the heat exchangers. The flow rate of the cooling water was controlled so that the liquid temperature (reaction temperature) in the reactor was maintained at 25°C. Note that the cooling water was not run through the jacket. The temperatures of the cooling water at the inlet and the outlet of the heat exchangers were 17°C and 22°C, respectively (ΔT = 5°C). A reaction from acrylonitrile to acrylamide was performed.

10 hours after the initiation of the reaction, the reaction solution (25°C) flowing out from the reactor was analyzed by means of gas chromatography and a refractometer. The acrylonitrile concentration was 0.4 wt%, and the acrylamide concentration was 11.0 wt% (ΔT/ΔC = 2.2). 95% or more of the acrylonitrile introduced into the reactor was converted into acrylamide.

### [Example 4]

### Reaction volute: 2 m³, double coil type heat exchanger, ΔT = 5°C

A reaction from acrylonitrile to acrylamide was performed in a manner similar to that in Example 3, except that the same double coil type heat exchangers as Example 2 were used.

10 hours after the initiation of the reaction, the reaction solution (25°C) flowing out from the reactor was analyzed by means of gas chromatography and a refractometer. The acrylonitrile concentration was 0.6 wt%, and the acrylamide concentration was 10.7 wt% (ΔC/ΔT = 2.1). 93% or more of the acrylonitrile introduced into the reactor was converted into acrylamide.

### [Comparative Example 3]

### External circulation type heat exchanger, ΔT = 5°C

A reaction from acrylonitrile to acrylamide was performed in a manner similar to that in Example 4, except that: the double coil type heat exchangers were removed from the reactor, and a circulation path of the reaction solution was provided to the outside of the reactor; a circulation pump and a fixed tube sheet type heat exchanger (outer diameter of a heat-transfer tube: 13.9 mm, length: 2.5 m, the number of heat-transfer tubes: 55, heat-transfer area: 6.0 m²) were provided to the circulation path; the reaction solution was flowed into the heat-transfer tubes; and cooling water was run at the shell side.

10 hours after the initiation of the reaction, the reaction solution (25°C) flowing out from the reactor was analyzed by means of gas chromatography and a refractometer. Since the biocatalyst was deactivated due to circulation of the reaction solution, the acrylonitrile concentration was 1.2 wt%, and the acrylamide concentration was 9.9 wt% (ΔC/ΔT = 2.0). Only 86% of the acrylonitrile supplied to the reactor was converted into acrylamide.

Further, costs for ancillary facilities such as pump and pipe arrangement for circulating the reaction solution and costs for circulation power were significantly increased compared to those in Examples 3 and 4.

### [Comparative Example 4]

### Redaction volume: 15 L, acrylamide roduction reaction using jacket-type heat exchanger

### Reactor with jacket made of SUS (inner diameter: 25 can, height: 40 cm, jacket heat-transfer area: 2360 cm²)

9.0 L/hr of 50 mM phosphate buffer (pH 7.0), 1.05 L/hr of acrylonitrile and 200 g/hr of diluted solution, in which the bacterial cell suspension produced in Example 1 was diluted 100-fold using 50 mM phosphate buffer, were continuously added, and the flow rate of discharging at the outlet of the reactor was controlled so that the liquid amount of the reaction mixture in the reactor became 15 L.

Cooling water was run through the jacket to control the temperature of the reaction mixture to become 25°C.

The temperatures of the cooling water at the inlet and the outlet of the jacket were set at 17°C and 22°C, respectively (ΔT = 5°C). 10 hours after the initiation of the reaction, the reaction solution (25°C) flowing out from the reactor was measured by means of gas chromatography and a refractometer. The acrylonitrile concentration was 0.3 wt%, and the acrylamide concentration was 11.1 wt% (ΔC/ΔT = 2.2). 95% or more of the acrylonitrile supplied to the reactor was converted into acrylamide.

Results regarding ΔT, temperature controllability, analysis of the reaction solution taken out from the reactor, etc. in the above-described working examples and comparative examples are shown in Table 1.

**[Table 1]**

| | Heat exchanger | Selling ΔT [°C] | Reaction scale | Temperature control | ΔC/ΔT [wt%/°C] | Rate of acrylamide conversion |
|---|---|---|---|---|---|---|
| Example 1 | Multitubular type | 20 | 2m³ | ○ | 1.7 | 95% or more |
| Example 2 | Double coil type | 20 | 2m³ | ○ | 1.7 | 95% or more |
| Example 3 | Multitubular type | 5 | 2m³ | ○ | 2.2 | 95% or more |
| Example 4 | Double coil type | 5 | 2m³ | ○ | 2.1 | 90% or more |
| Example 1 | Jacket cooling | 20 | 2m³ | × | 1.3 | 85% or more |
| Comparative Example 2 | Single coil type | 20 | 2m³ | × | 1.3 | 85% or more |
| Comparative Example 3 | External circulation type | 5 | 2m³ | ○ | 2.0 | 90% or more |
| Comparative Example 4 | Jacket cooling | 5 | 15L | ○ | 2.2 | 95% ormore |

As shown in Table 1, in Examples 1-4 according to the production method of the present invention, in the 2 m³ reaction that is an industrial scale, reaction heat was efficiently removed, and the rate of conversion from acrylonitrile to acrylamide was successfully 90% or more.

On the other hand, in Comparative Examples 1 and 2, in the 2 m³ reaction, reaction heat was not sufficiently removed, and the rate of conversion from acrylonitrile to acrylamide was decreased.

In Comparative Example 3, in the reaction of 2 m³ scale, reaction heat was successfully removed, but since the catalyst was deactivated due to circulation of the reaction solution, the rate of conversion from acrylonitrile to acrylamide was decreased. Further, costs for equipments for external circulation of the reaction solution and energy costs for circulation were increased, and costs for the production of acrylamide were significantly increased compared to Examples 3 and 4.

In Comparative Example 4, since it was a reaction at a scale of 15 L which is much smaller than the industrial scale, reaction heat was successfully removed even by jacket-type cooling.

### INDUSTRIAL APPLICABILITY

In the production method of the present invention, at the time of producing acrylamide at an industrial scale using a biocatalyst, reaction heat can be efficiently removed. Therefore, energy costs for cooling can be decreased, and the rate of conversion from acrylonitrile to acrylamide can be increased. Accordingly, it is possible to produce acrylamide with high productivity at low cost.

### EXPLANATIONS OF LETTERS OR NUMERALS

- 1: heat-transfer tube
- 2: reactor
- 3: division plate (the number of divisions: 3, the number of flow paths: 4)
- 4: fixing support plate
- 5: divided chamber
- 6: tube plate

## Claims

1. A method for producing acrylamide by reacting acrylonitrile in the presence of a biocatalyst, wherein the reaction is performed using a reactor having a reaction volume of 1 m³ or more equipped with a tubular heat exchanger in the inside of said reactor, and wherein the difference between a temperature of a cooling water to be introduced into the heat exchanger and a reaction temperature is maintained from 5 to 20°C.

2. The method according to claim 1, wherein the reaction is a continuous reaction.

3. The method according to claim 2, wherein the reaction is performed with a value that is obtained by dividing the difference between an acrylamide concentration in a reaction mixture at an inlet of the reactor and that at an outlet of the reactor (wt%) by the difference between the temperature of the cooling water and the reaction temperature (°C) being maintained within a range of 1.5 to 2.5.

4. The method according to any one of claims 1 to 3, wherein the tubular heat exchanger is a double or more coil type heat exchanger.

5. The method according to any one of claims 1 to 3, wherein the tubular heat exchanger is a multitubular heat exchanger.

6. The method according to claim 5, wherein the multitubular heat exchanger is a U-tube type heat exchanger.

7. The method according to claim 6, wherein the number of flow paths of the U-tube type heat exchanger is 2 to 6.

8. The method according to claim 6 or 7, wherein the U-tube type heat exchanger is installed upright in the reactor.

9. The method according to any one of claims 6 to 8, wherein the U-tube type heat exchanger has a structure in which a tube bundle thereof is detachable.

## Patentansprüche

1. Verfahren zur Herstellung von Acrylamid durch Umsetzen von Acrylonitril in der Gegenwart eines Biokatalysators, wobei die Reaktion unter Verwendung eines Reaktors mit einem Reaktorvolumen von 1 m³ oder mehr durchgeführt wird, der mit einem rohrförmigen Wärmetauscher im Inneren dieses Reaktors ausgestattet ist, und wobei die Differenz zwischen einer Temperatur eines Kühlwassers, das in den Wärmetauscher eingeführten werde soll, und einer Reaktionstemperatur bei 5 bis 20°C gehalten wird.

2. Verfahren gemäß Anspruch 1, wobei die Reaktion eine kontinuierliche Reaktion ist.

3. Verfahren gemäß Anspruch 2, wobei die Reaktion mit einem Wert durchgeführt wird, der durch Dividieren der Differenz zwischen einer Acrylamidkonzentration in einer Reaktionsmischung an einem Einlass des Reaktors und der an einem Auslass des Reaktors (Gew.%) durch die Differenz zwischen der Temperatur des Kühlwassers und der Reaktionstemperatur (°C) erhalten wird, wobei der Wert innerhalb eines Bereiches von 1,5 bis 2,5 gehalten wird.

4. Verfahren gemäß einem der Ansprüche 1 bis 3, wobei der rohrförmige Wärmetauscher ein Wärmetauscher vom Doppel- oder Mehrspiralen-Typ ist.

5. Verfahren gemäß einem der Ansprüche 1 bis 3, wobei der rohrförmige Wärmetauscher ein Rohrbündelwärmetauscher ist.

6. Verfahren gemäß Anspruch 5, wobei der Rohrbündelwärmetauscher ein Wärmetauscher vom U-Rohrtyp ist.

7. Verfahren gemäß Anspruch 6, wobei die Anzahl der Strömungswege des U-Rohrtyp-Wärmetauschers 2 bis 6 ist.

8. Verfahren gemäß Anspruch 6 oder 7, wobei der U-Rohrtyp-Wärmetauscher in dem Reaktor aufrecht installiert ist.

9. Verfahren gemäß einem der Ansprüche 6 bis 8, wobei der U-Rohrtyp-Wärmetauscher einen Aufbau besitzt, in dem ein Rohrbündel davon abnehmbar ist.

## Revendications

1. Procédé de production d'acrylamide en faisant réagir de l'acrylonitrile en présence d'un biocatalyseur, dans lequel la réaction est réalisée en utilisant un réacteur ayant un volume de réaction de 1 m³ ou plus équipé avec un échangeur de chaleur tubulaire à l'intérieur dudit réacteur, et dans lequel la différence entre une température d'une eau de refroidissement destinée à être introduite dans l'échangeur de chaleur et une température de réaction est maintenue de 5 à 20°C.

2. Procédé selon la revendication 1, dans lequel la réaction est une réaction continue.

3. Procédé selon la revendication 2, dans lequel la réaction est réalisée avec une valeur, qui est obtenue en divisant la différence entre une concentration d'acrylamide dans un mélange réactionnel à une entrée du réacteur et celle à une sortie du réacteur (% en poids) par la différence entre la température de l'eau de refroidissement et la température de réaction (°C), étant maintenue dans une plage de 1,5 à 2,5.

4. Procédé selon l'une quelconque des revendications 1 à 3, dans lequel l'échangeur de chaleur tubulaire est un échangeur de chaleur du type à deux bobines ou plus.

5. Procédé selon l'une quelconque des revendications 1 à 3, dans lequel l'échangeur de chaleur tubulaire est un échangeur de chaleur multitubulaire.

6. Procédé selon la revendication 5, dans lequel l'échangeur de chaleur multitubulaire est un échangeur de chaleur du type à tubes en U.

7. Procédé selon la revendication 6, dans lequel le nombre de chemins d'écoulement de l'échangeur de chaleur du type à tubes en U est 2 à 6.

8. Procédé selon la revendication 6 ou 7, dans lequel l'échangeur de chaleur du type à tubes en U est installé de manière verticale dans le réacteur.

9. Procédé selon l'une quelconque des revendications 6 à 8, dans lequel l'échangeur de chaleur du type à tubes en U a une structure dans laquelle un faisceau de tubes de celui-ci est détachable.
